(19) Europäisches Patentamt
European Patent Office
Office européen des brevets

(11) **EP 3 356 514 B1**

(12) **FASCICULE DE BREVET EUROPEEN**

(45) Date de publication et mention de la délivrance du brevet:
**07.06.2023 Bulletin 2023/23**

(21) Numéro de dépôt: **16785206.0**

(22) Date de dépôt: **22.09.2016**

(51) Classification Internationale des Brevets (IPC):
**C12N 1/21** *(2006.01)*   **C12N 9/00** *(2006.01)*
**C12N 9/88** *(2006.01)*   **C12N 15/74** *(2006.01)*
**C12P 7/24** *(2006.01)*

(52) Classification Coopérative des Brevets (CPC):
**C12N 9/88; C12N 9/93; C12N 15/74; C12P 7/24;
C12Y 401/02041; C12Y 402/01101;
C12Y 602/01034**

(86) Numéro de dépôt international:
**PCT/FR2016/052403**

(87) Numéro de publication internationale:
**WO 2017/055712 (06.04.2017 Gazette 2017/14)**

(54) **NOUVELLES SOUCHES BACTERIENNES POUR LA PRODUCTION DE VANILLINE**

NEUARTIGE BAKTERIENSTÄMME ZUR HERSTELLUNG VON VANILLIN

NOVEL BACTERIAL STRAINS FOR THE PRODUCTION OF VANILLIN

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **29.09.2015 FR 1559142**

(43) Date de publication de la demande:
**08.08.2018 Bulletin 2018/32**

(73) Titulaire: **Lesaffre et Compagnie
75001 Paris (FR)**

(72) Inventeurs:
• **DARRICAU, Mylène
64300 Balanson (FR)**
• **DESFOUGERES, Thomas
59960 Neuville En Ferrain (FR)**
• **PERNODET, Jean-Luc
94230 Cachan (FR)**

(74) Mandataire: **Plasseraud IP
31, rue des Poissonceaux
CS 40009
59044 Lille Cedex (FR)**

(56) Documents cités:
**WO-A1-2012/172108   WO-A1-2015/066722
WO-A2-01/44480**

**Description**

DOMAINE DE L'INVENTION

La présente invention concerne le domaine de la production de la vanilline. Elle concerne particulièrement une souche *Amycolatopsis* dérivée de la souche *Amycolatopsis* Zyl 926.

ARRIÈRE PLAN TECHNIQUE

La vanilline est l'un des composants majeur constituant l'extrait de vanille qui est un puissant arôme naturel obtenu à partir de la gousse d'une orchidée. L'extraction de cette molécule à partir de gousses de vanille est très onéreuse, les quantités produites sont limitées et ne couvrent pas la demande du marché. D'autres voies d'obtention de la vanilline ont ainsi été recherchées.

Il est par exemple possible d'obtenir de la vanilline à partir d'acide férulique par une réaction de bioconversion grâce à des bactéries. Dans la présente demande de brevet nous nous intéressons uniquement à la voie de bioconversion de l'acide férulique en vanilline par les bactéries.

La production de vanilline par des bactéries se fait par déacétylation de l'acide férulique au moyen des 3 étapes suivantes :

1. activation de l'acide férulique en feruloyl-CoA, l'activation étant catalysée par une enzyme, la Feruloyl-CoA Synthétase, ci-après FCS ;
2. hydroxylation de la Feruloyl-CoA en 4-Hydroxy-3-methoxyphenyl-$\beta$-hydroxypropionyl-CoA, l'hydroxylation étant catalysée par une enzyme, l'Enoyl-CoA-Hydratase/Aldolase, ci-après ECH ;
3. déacétylation du 4-Hydroxy-3-methoxyphenyl-$\beta$-hydroxypropionyl-CoA permettant l'obtention de vanilline, catalysée également par l'enzyme ECH.

La vanilline obtenue peut ensuite être oxydée en acide vanillique par une enzyme, la Vanilline DéHydrogénase, ci-après VDH. Toutefois la conversion de la vanilline en acide vanillique n'est pas souhaitée car seule la vanilline présente les caractéristiques aromatiques recherchées.

Selon la nomenclature EC des enzymes (Enzyme Commission number) :

- l'enzyme FCS a le code EC 6.2.1.34,
- l'enzyme ECH a les codes EC 4.2.1.101 et EC 4.1.2.41,
- l'enzyme VDH a le code EC 1.2.1.67.

EP 2 721 148 A1, équivalent à WO 2012/172108 A1, décrit un microorganisme du genre *Amycolatopsis* ne comprenant pas le gène codant la *vdh,* ledit microorganisme permettant de produire de la vanilline à partir d'acide férulique.

EP 1 611 244 A1 décrit un procédé de production de vanilline n'utilisant pas de solvants organiques car ils sont désormais considérés comme non souhaitables dans l'industrie agro-alimentaire, lors de la purification afin d'obtenir une vanilline dite « naturelle ». Au cours dudit procédé de production de vanilline la biotransformation de l'acide férulique en vanilline est réalisée par la souche *Amycolatopsis* IMI390106, appelée également souche Zyl 926 ou souche *Amycolatopsis* Zyl 926.

Le livre « Practical Streptomyces Genetics », 2000, Kieser, et al. publié par The John Innes Foundation, ISBN 0-7084-0623-8, dans son chapitre 10, décrit une méthode de conjugaison entre *Escherichia coli* et *Streptomyces* permettant l'intégration d'ADN au niveau de sites spécifiques.

Toutefois, les souches de l'art antérieur utilisées lors de la réaction de bioconversion ne permettent pas une conversion optimale de l'acide férulique en vanilline. Etant donné le coût de l'acide férulique, il existe toujours un besoin de nouvelles souches de bactéries assurant une meilleure conversion de l'acide férulique en vanilline.

RESUME DE L'INVENTION

Le demandeur a ainsi développé de nouvelles souches de bactéries telles que revendiquées, grâce à un procédé de transformation original, permettant d'avoir un rendement molaire amélioré lors de la réaction de bioconversion.

L'invention a pour objet une souche *Amycolatopsis sp.* déposée auprès de la CNCM sous le numéro I-4922, I-4923 ou I-4924 le 11 décembre 2014.

L'invention a également pour objet l'utilisation d'une souche *Amycolatopsis sp.* telle que définie ci-dessus dans un procédé d'obtention de la vanilline.

L'invention a également pour objet un procédé de production de la vanilline, ledit procédé étant caractérisé en ce que la réaction de bioconversion est réalisée dans un milieu comprenant une souche *Amycolatopsis sp.* telle que définie ci-dessus.

DESCRIPTION DETAILLEE

Il est décrit dans la présente demande une souche *Amycolatopsis* dérivée de la souche *Amycolatopsis* Zyl 926, possédant au moins une copie supplémentaire des gènes *fcs* et *ech* intégrée au niveau du site d'intégration du phage φC31.

Il est également décrit un procédé d'obtention d'une souche *Amycolatopsis* dérivée de la souche *Amycolatopsis* Zyl 926 par conjugaison interspécifique.

Il est encore décrit une cassette d'intégration comprenant la cassette d'expression des gènes *ech* et *fcs.*

La présente invention a pour objet de nouvelles souches de bactéries du genre *Amycolatopsis* telles que revendiquées utilisables lors de la réaction de bioconversion de l'acide férulique en vanilline.

Les inventeurs ont développé après de longues recherches de nouvelles souches *Amycolatopsis* telles que revendiquées dérivées de la souche *Amycolotapsis* Zyl 926 permettant d'avoir un rendement molaire amélioré par rapport à l'état de la technique, lors de la réaction de bioconversion de l'acide férulique en vanilline grâce à la surexpression des gènes *fcs* et *ech* dans le génome de la souche *Amycolotapsis* Zyl 926.

Un rendement molaire de bioconversion se définit comme étant le rapport entre le nombre de moles de produit obtenu, dans notre cas la vanilline, divisé par le nombre de moles de substrat mis en oeuvre, dans notre cas l'acide férulique. Lorsqu'il est sous forme de pourcentage ce rapport est multiplié par 100.

Par « rendement molaire amélioré par rapport à l'état de la technique », on entend un rendement molaire de bioconversion supérieur ou égal à 80%, de préférence supérieur ou égal à 85%, et encore plus de préférence égal à 90%.

La souche *Amycolatopsis* Zyl 926 a été déposée au CABI Bioscience, UK science centre à Egham, sous le numéro IMI 390106 le 2 mars 2003.

Les termes « souche Zyl 926 », « souche *Amycolatopsis* Zyl 926 », « souche *Amycolatopsis* IMI 390106 », « souche *Amycolatopsis* déposée au CABI Bioscience sous le numéro IMI 390106 » sont ici synonymes.

Par « souche(s) *Amycolatopsis* dérivée(s) de la souche *Amycolotapsis* Zyl 926 », on entend toutes souches obtenues après transformation de la souche Zyl 926 par conjugaison interspécifique permettant l'intégration d'au moins une copie supplémentaire des gènes *fcs* et *ech.*

Les inventeurs ont en outre mis en évidence que les nouvelles souches *Amycolotapsis* telles que revendiquées permettent également d'améliorer la productivité et la consommation de la quasi-totalité de l'acide férulique lors de la réaction de bioconversion.

Par « consommation de la quasi-totalité de l'acide férulique », on entend qu'au moins 98% de l'acide férulique soit consommé lors de la réaction de bioconversion, de préférence au moins 99%, et encore plus de préférence 100%.

Il est décrit dans la présente demande une souche *Amycolatopsis* dérivée de la souche *Amycolatopsis* Zyl 926 comprenant :

- au moins une copie supplémentaire du gène *fcs* codant la Feruloyl-coA-Synthétase ayant pour séquence la séquence protéique SEQ ID NO : 1 ou toute séquence identique à au moins 80%, de préférence au moins 90%, et encore plus de préférence au moins 95% à la séquence SEQ ID NO : 1, et

- au moins une copie supplémentaire du gène ech codant l'Enoyl-coA-Hydratase/Aldolase ayant pour séquence la séquence protéique SEQ ID NO : 2 ou toute séquence identique à au moins 80%, de préférence au moins 90%, et encore plus de préférence au moins 95% à la séquence SEQ ID NO : 2,

lesdites copies supplémentaires des gènes *fcs* et *ech* étant intégrées spécifiquement au niveau du site d'intégration du phage cpC31, également appelé site *attB.*

L'article de Keravala et al. (Methods Mol Biol. 2008) mentionne l'utilisation de l'intégrase du phage φC31 comme médiateur pour une intégration chromosomique site spécifique. Elle permet ainsi d'effectuer une recombinaison unidirectionnelle entre deux sites *att* appelés *attP* et *attB.*

Par « copie supplémentaire du gène », on entend toute copie d'un gène d'intérêt intégrée dans le génome d'une souche en plus des copies du gène d'intérêt présentes initialement dans le génome.

Une séquence protéique ou nucléique est dite identique à une autre lorsque l'alignement entre les deux séquences est parfait c'est-à-dire lorsqu'il y a une parfaite correspondance respectivement entre les acides aminés ou acides nucléiques de chaque séquence.

Le pourcentage d'identité de séquence se calcule en donnant un score de 0 ou 1 pour chaque acide aminé ou base de la séquence selon qu'il y ait identité ou non.

Les expressions « gène *ech* codant l'Enoyl-coA-Hydratase/Aldolase » et « gène *fcs* codant la Feruloyl-coA-Synthétase » ne doivent pas être interprétées de manière stricte mais elles doivent englober également les séquences codant les variants fonctionnels de ces enzymes. Typiquement, des variants fonctionnels de ces enzymes FCS et ECH ont une séquence protéique présentant un pourcentage d'identité d'au moins 80%, de préférence d'au moins 90% et plus particulièrement de préférence d'au moins 95% avec les séquences protéiques SEQ ID NO : 1 et SEQ ID NO : 2.

De façon avantageuse, les copies supplémentaires des gènes *fcs* et *ech* de la souche *Amycolatopsis* sont placées sous la dépendance du promoteur fort ermE* associé à un site d'association du ribosome, appelé RBS.

Le promoteur ermE* correspond au promoteur ermE modifié de façon à lui donner une expression forte et constitutive.

Le promoteur ermE* a pour séquence la séquence nucléique SEQ ID NO : 6 ou toute séquence identique à au moins 90%, de préférence au moins 95% et encore plus de préférence au moins 99% à la séquence SEQ ID NO : 6.

La séquence du promoteur ermE* est celle décrite dans l'article de Bibb et al. (Mol Microbiol. 1994 Nov;14(3):533-45)

Pour obtenir de nouvelles souches originales telles que revendiquées, surexprimant les gènes *ech* et *fcs,* les inventeurs ont mis en évidence après de nombreuses recherches et expérimentations que la méthode de conjugaison interspécifique permettait d'obtenir des transformants ayant intégré au moins une copie supplémentaire des gènes *ech* et *fcs* et que la réussite de cette méthode d'intégration de gènes était locus dépendante.

On entend par « locus dépendante », le fait que les copies supplémentaires des gènes d'intérêt doivent être intégrées spécifiquement au niveau du site d'intégration du phage φC31.

Il est encore décrit dans la présente demande un procédé d'obtention d'une souche *Amycolatopsis* dérivée de la souche *Amycolatopsis* Zyl 926 telle que décrite ci-dessus, comprenant les étapes suivantes :

a. amplification par PCR des gènes *fcs* codant la Feruloyl-coA-Synthétase ayant pour séquence la séquence SEQ ID NO : 1 ou toute séquence identique à au moins 80%, de préférence au moins 90%, et encore plus de préférence au moins 95% à la séquence SEQ ID NO : 1 et *ech* codant l'Enoyl-coA-Hydratase/Aldolase ayant pour séquence la séquence SEQ ID NO : 2 ou toute séquence identique à au moins 80%, de préférence au moins 90%, et encore plus de préférence au moins 95% à la séquence SEQ ID NO : 2, isolés d'une souche *Amycolatopsis;*

b. construction d'un vecteur portant les séquences nucléiques obtenues à l'étape a., à partir du plasmide pSET152 possédant :

i. le gène *int* codant l'intégrase du phage φC31 ayant pour séquence la séquence nucléotidique SEQ ID NO : 3 ou toute séquence identique à au moins 80%, de préférence au moins 90%, et encore plus de préférence au moins 95% de la séquence SEQ ID NO : 3,le site de fixation du phage φC31, ci-après site *attP* ayant pour séquence la séquence nucléotidique SEQ ID NO : 4, et

iii. l'origine de conjugaison *oriT* fonctionnelle ayant pour séquence la séquence nucléotidique SEQ ID NO : 5 ou toute séquence identique à au moins 90%, de préférence au moins 95% et encore plus de préférence au moins 99% à la séquence SEQ ID NO : 5 ;

c. transformation par électroporation de la souche *Escherichia coli* ET12567 avec un plasmide conjuguant comprenant tous les gènes codant la machinerie de conjugaison dont l'origine de conjugaison *oriT* rendue non fonctionnelle, et le vecteur construit à l'étape b. ;

d. sélection des souches *Escherichia coli* ET12567 transformées à l'étape c. par culture sur un milieu contenant la néomycine et une bêta-lactamine ;

e. conjugaison interspécifique entre les souches *Escherichia coli* ET12567 transformées et sélectionnées aux étapes c. et d. et la souche *Amycolatopsis* Zyl 926, par mise en co-culture ;

f. sélection des souches *Amycolatopsis* Zyl 926 ayant intégré au moins une copie supplémentaire du gène *fcs* codant la Feruloyl-coA-Synthétase ayant pour séquence la séquence SEQ ID NO : 1 ou toute séquence identique à au moins 80%, de préférence au moins 90%, et encore plus de préférence au moins 95% à la séquence SEQ ID NO : 1 et au moins une copie supplémentaire du gène *ech* codant l'enoyl-coa-hydratase ayant pour séquence la séquence SEQ ID NO : 2 ou toute séquence identique à au moins 80%, de préférence au moins 90%, et encore plus de préférence au moins 95% à la séquence SEQ ID NO : 2 selon l'étape e., par culture sur un milieu contenant de l'apramycine.

Les gènes *fcs* et *ech* ont été isolés et amplifiés à partir d'une souche *Amycolatopsis* par une technique PCR qui est une technique connue de l'homme du métier. En raison des particularités propres au génome *d'Amycolatopsis,* le kit « Phusion® High-Fidelity PCR Master Mix with GC Buffer » de New England Biolabs a été préférentiellement utilisé avec le tampon GC Buffer.

Les séquences des amorces utilisées sont les séquences suivantes :

- SEQ ID NO : 7 : Van-ECH-F : 5' GAAGCTTGAGCGATGCATGAGCACAGC 3'
- SEQ ID NO : 8 : Van-ECH-R : 5' GTCTAGACTGGTTGCGCACTACTTCTC 3'

De manière préférentielle les gènes *fcs* et *ech* ont été isolés de la souche *Amycolatopsis* Zyl 926.

Le plasmide pSET152 est porteur de la résistance à l'apramycine.

La construction du vecteur selon l'étape b. est réalisée par clonage des copies supplémentaires des gènes *fcs* et *ech* amplifiés à l'étape a. dans le plasmide pSET152 (Bierman et al., Gene, 116 (1) : 43-49, 1992). Le produit de PCR contenant les gènes *ech* et *fcs* a été obtenu en utilisant comme matrice l'ADN génomique de la souche Zyl926 et les amorces Van-ECH-F (SEQ ID N0 : 7) et Van-ECH-R (SEQ ID N0 : 8). Le fragment à intégrer a été obtenu par digestion HindIII (+ Klenow) et XbaI. Les fragments HindIII (+ Klenow) et XbaI ont été clonés dans les sites EcoRV et XbaI de pSET152.

Par gène *int,* on entend le gène codant l'intégrase du phage φC31 ayant pour séquence la séquence protéique SEQ ID NO : 3 ou toute séquence identique à au moins 80%, de préférence au moins 90%, et encore plus de préférence au moins 95% de la séquence SEQ ID NO : 3.

Le site de fixation du phage cpC31, également appelé site *attP,* présent dans le plasmide pSET152, a pour séquence la séquence SEQ ID NO : 4.

La séquence SEQ ID NO : 4 est la suivante :

GCCCCAACTGGGGTAACCTTTGAGTTCTCTCAGTTGGGG

La sélection des souches *Escherichia coli* ET 12567 transformées à l'étape c est réalisée sur la base de l'acquisition par ces souches transformées de la résistance à la néomycine et à une bêta-lactamine.

La mise en co-culture, pour la réalisation de la conjugaison interspécifique de l'étape e., est réalisée dans des conditions classiques connues de l'homme du métier (Bierman et al., 1992 cf. *supra*) et préférentiellement par mise en contact durant plusieurs heures d'une part des *Escherichia coli* ET12567 obtenues à l'étape d. transformées préalablement et rendues compétentes pour la transformation, et d'autre part des cellules *d'Amycolatopsis* Zyl 926 préalablement synchronisées pour la germination par chauffage.

La souche *Escherichia coli* ET12567 est la souche ATCC BAA-525 (MacNeil et al., Gene 111: 61-68, 1992).

La sélection selon l'étape f. des souches *Amycolatopsis* Zyl 926 ayant intégré au moins une copie supplémentaire des gènes *fcs* et *ech* est réalisée sur la base de l'acquisition par ces souches de la résistance à l'apramycine.

Selon un mode particulier de réalisation, le procédé d'obtention d'une souche *Amycolatopsis* dérivée de la souche *Amycolatopsis* Zyl 926 tel que décrit ci-dessus, comprend une étape supplémentaire b'., entre les étapes b. et c., d'amplification et de clonage du promoteur fort ermE* dans le vecteur construit à l'étape b., ledit promoteur ermE* étant associé à un site d'association du ribosome, appelé RBS.

Le promoteur ermE* a pour séquence la séquence SEQ ID NO : 6 ou toute séquence identique à au moins 90%, de préférence au moins 95% et encore plus de préférence au moins 99% à la séquence SEQ ID NO : 6.

Selon un mode particulier de réalisation du procédé d'obtention d'une souche *Amycolatopsis* dérivée de la souche *Amycolatopsis* Zyl 926 tel que décrit ci-dessus, le plasmide conjuguant de l'étape c. est le plasmide pUZ8002 décrit dans le livre « Practical Streptomyces Genetics », 2000, Kieser, et al., chapitre 10, publié par The John Innes Foundation, ISBN 0-7084-0623-8.

Selon un mode particulier de réalisation du procédé d'obtention d'une souche *Amycolatopsis* dérivée de la souche *Amycolatopsis* Zyl 926 tel que décrit ci-dessus, la bêta-lactamine de l'étape d. est l'ampicilline.

Il est encore décrit dans la présente demande une souche *Amycolatopsis* dérivée de la souche *Amycolatopsis* Zyl 926 susceptible d'être obtenue par le procédé tel que défini ci-dessus.

La présente invention a particulièrement pour objet une souche *Amycolatopsis* dérivée de la souche *Amycolatopsis* Zyl 926, susceptible d'être obtenue par le procédé tel que défini ci-dessus, qui est choisie parmi la souche déposée à la CNCM sous le numéro I-4922, la souche déposée à la CNCM sous le numéro I-4923, et la souche déposée à la CNCM sous le numéro I-4924.

Les souches I-4922, I-4923 et I-4924 sont des souches *Amycolatopsis* sp. déposées en vertu du Traité de Budapest le 11 décembre 2014 auprès de la CNCM (*Collection Nationale de Cultures de Microorganismes*)*,* 25 rue du Docteur Roux, 75724 Paris cedex 15, France.

Il est encore décrit dans la présente demande une cassette d'intégration comprenant :

i. une cassette d'expression des gènes *fcs* codant la Feruloyl-coA-Synthétase ayant pour séquence la séquence SEQ ID NO : 1 ou toute séquence identique à au moins 80%, de préférence au moins 90%, et encore plus de préférence au moins 95% à la séquence SEQ ID NO : 1 et *ech* codant l'Enoyl-coA-Hydratase/Aldolase ayant pour séquence la séquence SEQ ID NO : 2 ou toute séquence identique à au moins 80%, de préférence au moins 90%, et encore plus de préférence au moins 95% à la séquence SEQ ID NO : 2 ;

ii. des moyens permettant l'intégration de la cassette dans le génome de la souche *Amycolatopsis* Zyl 926, comprenant le gène *int* du phage φC31 ayant pour séquence la séquence SEQ ID NO : 3 ou toute séquence identique à au moins 80%, de préférence au moins 90%, et encore plus de préférence au moins 95% de la séquence SEQ ID NO : 3 et le site de fixation du phage φC31, appelé site *attP* ayant pour séquence la séquence SEQ ID NO : 4 ;

iii. des moyens de sélection des souches *Escherichia coli* ET12567 transformées comprenant la néomycine et une bêta-lactamine ;

iv. des moyens de sélection des souches *Amycolatopsis* Zyl926 ayant intégré ladite cassette d'intégration, comprenant un marqueur de résistance à l'apramycine.

Une cassette d'intégration est une construction génétique comprenant les outils nécessaires à l'intégration d'un ou plusieurs gènes d'intérêt dans le génome d'un organisme, ici la souche *Amycolatopsis* Zyl 926.

Selon un mode particulier de réalisation, les gènes codant la Feruloyl-coA-Synthétase et l'Enoyl-coA-Hydratase/Aldolase de la cassette d'expression décrite ci-dessus sont placés sous la dépendance du promoteur fort ermE* ayant pour séquence la séquence SEQ ID NO : 6 ou toute séquence identique à au moins 90%, de préférence au moins 95% et encore plus de préférence au moins 99% à la séquence SEQ ID NO : 6 ledit promoteur ermE* étant associé à un site d'association du ribosome, également appelé RBS.

Selon un mode particulier de réalisation de la cassette d'intégrationtelle que décrite ci-dessus, la bêta-lactamine est l'ampicilline.

L'invention a également pour objet l'utilisation des nouvelles souches *Amycolatopsis sp.* dérivées de la souche *Amycolatopsis* Zyl 926 telles que décrites ci-dessus, dans un procédé de production de vanilline.

Il est également décrit dans la présente demande un procédé de production de vanilline comprenant les étapes suivantes :

a. disposer d'une souche *Amycolatopsis* dérivée de la souche *Amycolatopsis* Zyl 926 déposée au CABI Bioscience sous le numéro IMI 390106 le 2 mars 2003, comprenant au moins une copie supplémentaire des gène *fcs* codant la Feruloyl-coA-Synthétase ayant pour séquence la séquence SEQ ID NO : 1 ou toute séquence identique à au moins 80%, de préférence au moins 90%, et encore plus de préférence au moins 95% à la séquence SEQ ID NO : 1. et *ech* codant l'Enoyl-coA-Hydratase/Aldolase ayant pour séquence la séquence SEQ ID NO : 2 ou toute séquence identique à au moins 80%, de préférence au moins 90%, et encore plus de préférence au moins 95% à la séquence SEQ ID NO : 2 et dont lesdites copies supplémentaires sont intégrées spécifiquement au niveau du site d'intégration du phage φC31 ;

b. mettre en culture la nouvelle souche *Amycolatopsis* dérivée de l'étape a. ;

c. mettre en présence l'acide férulique avec ladite culture de la souche *Amycolatopsis* de l'étape b. et incuber ;

d. extraire la vanilline du milieu de culture.

La mise en culture des nouvelles souches *Amycolatopsis* dérivées est effectuée préférentiellement sur des milieux contenant tous les nutriments nécessaires à la bonne croissance de la bactérie, notamment une source de carbone, à des températures et pH régulés dans des fermenteurs.

Une source de carbone peut par exemple être du glycérol.

L'incubation selon l'étape c. du procédé de production de vanilline est réalisée jusqu'à la consommation de la quasi-totalité de l'acide férulique et à une température comprise entre 37°C et 41°C.

Selon un mode de réalisation particulier le procédé de production de vanilline est le procédé décrit dans la demande EP 1 611 244 A1.

La réaction de bioconversion lors du procédé de production de vanilline est donc réalisée dans un milieu comprenant une nouvelle souche *Amycolatopsis* sp. dérivée de la souche *Amycolatopsis* Zyl 926 selon l'invention.

Selon un mode particulier de réalisation de l'invention, la souche *Amycolatopsis* dérivée de la souche *Amycolatopsis* Zyl 926 mise en oeuvre dans le procédé de production de vanille à l'étape a., est choisie parmi la souche déposée à la CNCM sous le numéro I-4922, la souche déposée à la CNCM sous le numéro I-4923, et la souche déposée à la CNCM sous le numéro I-4924.

Le procédé de production de vanilline tel que décrit ci-dessus a un rendement molaire supérieur ou égal à 80%, de préférence supérieur ou égal à 85%, et encore plus de préférence égal à 90%.

Dans le procédé de production de vanilline tel que décrit ci-dessus, au moins 98% de l'acide férulique est consommé, de préférence au moins 99%, et encore plus de préférence 100%.

Les inventeurs mettant en oeuvre ledit procédé de production de vanilline tel que décrit ci-dessus avec les nouvelles souches *Amycolatopsis* telles que revendiquées ont ainsi remarqué de manière étonnante que :

- la quasi-totalité de l'acide férulique est consommée lors de la réaction de bio conversion ;
- la productivité est améliorée;

- le rendement molaire est amélioré ;

et cela sans que le flux de vanilline déshydrogénase transformant la vanilline en acide vanillique ne soit augmenté.

Il est encore décrit dans la présente demande une nouvelle souche *Amycolatopsis* telle que décrite ci-dessus dont le gène *vdh* codant la Vanilline DésHydrogénase n'est pas supprimé. Il est également décrit dans la présente demande un procédé d'obtention d'une nouvelle souche *Amycolatopsis* tel que décrit ci-dessus ne comportant pas d'étape de suppression du gène *vdh* codant la Vanilline DésHydrogénase.

EXEMPLES

EXEMPLE 1 : Clonage de copies supplémentaires des gènes *ech* et *fcs* dans Zyl 926 au niveau du site d'intégration du phage φC31

*Matériel et méthode*

Souches bactériennes :

- *Escherichia coli* ET12567
- *Amycolatopsis* Zyl 926

Plasmides :

- p SET152 : porteur d'un gène de résistance à l'ampicilline
- pUZ8002 : porteur d'un gène de résistance à la néomycine

a. amplification

Les gènes *ech* et *fcs* ont été amplifiés à partir de la souche *Amycolatopsis* Zyl 926 en utilisant le kit « Phusion® High-Fidelity PCR Master Mix with GC Buffer » avec un tampon GC Buffer.

Les amorces utilisées sont les suivantes :

- SEQ ID N0 : 7 : Van-ECH-F : 5' GAAGCTTGAGCGATGCATGAGCACAGC 3'

- SEQ ID N0 : 8 : Van-ECH-R : 5' GTCTAGACTGGTTGCGCACTACTTCTC 3'

b. clonage

Le fragment à intégrer est obtenu par digestion HindIII (+ Klenow) et XbaI.

Le fragment HindIII (+ Klenow) et XbaI est ensuite cloné dans les sites EcoRV et XbaI de pSET152. Le plasmide ainsi obtenu est appelé pLSF01a.

Le plasmide pSET152 possède le système d'intégration du phage φC31 à savoir :

- le gène *int* codant l'intégrase du phage φC31 ;
- le site *attP* de fixation phage φC31 ;

Le plasmide pSET152 possède également l'origine de conjugaison *oriT* fonctionnelle permettant sa mobilisation et son transfert de *E. coli* à *Amycolatopsis* par la souche *E. coli* ET12567 + pUZ8002.

b'. amplification et clonage du promoteur ermE*

L'ajout du promoteur ermE* et du gène de résistance au chloramphénicol a été réalisé par recombinaison *in vivo* entre de courtes séquences identiques (PCR-targeting) comme décrit par Yu et al. (2000) (Proc Natl Acad Sci USA. 2000 May 23;97(11):5978-83).

Pour cela une séquence correspondant au gène de résistance au chlroramphénicol, au promoteur *ermE*\* et au RBS du gène *tipA* a été obtenue par PCR en utilisant comme matrice le plasmide pOSV605 et comme amorces les oligonucléotides LG001 et LG002.Les séquences des amorces utilisées sont les suivantes :

- SEQ ID N0 : 9 : LG001 :

5' CAGCTATGACATGATTACGAATTCGATAGCTTAGCGATGCTCACGCAGTTA
GACACTCAC 3'

- SEQ ID N0 : 10 : LG002 :

5' GCTCCGTCCGGACCCGCCCGTTGCCGACCGCTGTGCTCATATGTCCGCTC
CCTTCTCCCGCGAATTCACTAGTGATT 3'

Ceci a permis d'obtenir un fragment présentant à chaque extrémité des séquences identiques à celles de deux régions proches sur le plasmide pLSF01a :

- l'une dans la partie du vecteur en amont du gène *ech,*
- l'autre à l'extrémité 5' du gène *ech.*

La recombinaison a eu lieu in vivo dans la souche DY330 (Yu et al. 2000) contenant le plasmide pLSF01, après transformation par le produit PCR.

Les clones ayant intégré le promoteur ermE* ont été sélectionnés sur la base de l'acquisition de la résistance au chloramphénicol. Les clones sélectionnés sont appelés plasmides pLSF02.

c. transformation

La souche ET12567 contenant le plasmide pUZ8002 est transformée par électroporation avec le plasmide pLSF02issu des étapes b. et b'., comprenant les copies supplémentaires des gènes *ech* et *fcs* et le promoteur fort ermE* associé au RBS.

d. sélection des transformants

La sélection des transformants possédant le plasmide pLSF02 se fait sur un milieu contenant de l'apramycine.

e. conjugaison interspécifique

Les cellules *d'Amycolatopsis* Zyl 926 sont synchronisées pour la germination par un chauffage à 50°C pendant 10 minutes.

Les transformants (*E. coli* ET12567+ pUZ8002+pLSF02) et les cellules *d'Amycolatopsis* Zyl 926 ainsi préparées sont ensuite mis en contact par une co-culture durant plusieurs heures pour la réalisation de la conjugaison interspécifique.

f. sélection des souches Zyl 926 transformées

La sélection des souches *Amycolatopsis* Zyl 926 ayant intégré des copies supplémentaires des gènes *ech* et *fcs* se fait sur un milieu contenant de l'apramycine.

*Résultats*

Cette dernière étape a permis notamment de sélectionner les souches déposée à la CNCM sous les numéros I-4922, I-4923, et I-4924 ayant intégré les copies supplémentaires des gènes *ech* et *fcs* au niveau du site d'intégration du phage φC31.

EXEMPLE 2 : Clonage de copies supplémentaires des gènes *ech* et *fcs* dans Zyl 926 au niveau du site d'intégration du phage φBT1

*Matériel et méthode*

Souches bactériennes :

- *Escherichia coli* ET12567
- *Amycolatopsis* Zyl 926

Plasmides :

- pOSV408 : porteur d'un gène de résistance à la kanamycine
- pUZ8002 : porteur d'un gène de résistance à la néomycine

a. amplification

Les gènes *ech* et *fcs* ont été amplifiés à partir de la souche *Amycolatopsis* Zyl 926 en utilisant le kit « Phusion® High-Fidelity PCR Master Mix with GC Buffer » avec un tampon GC Buffer.
Les amorces utilisées sont :

- SEQ ID N0 : 7 : Van-ECH-F : 5' GAAGCTTGAGCGATGCATGAGCACAGC 3'

- SEQ ID N0 : 8 : Van-ECH-R : 5' GTCTAGACTGGTTGCGCACTACTTCTC 3'

b. clonage

Le fragment à intégrer est obtenu par digestion HindIII (+ Klenow) et XbaI.
Le fragment HindIII (+ Klenow) et XbaI est ensuite cloné dans les sites EcoRV et XbaI de pOSV408. Le plasmide ainsi obtenu est appelé pLSF01b
Le plasmide pOSV408 (KanaR) possède le système d'intégration du phage φBT1 à savoir :

- le gène *int* codant l'intégrase du phage φBT1 ;
- le site *attP* de fixation phage φBT1 ;

Le plasmide pOSV408 possède également l'origine de conjugaison *oriT* fonctionnelle permettant sa mobilisation et son transfert de *E. coli* à *Amycolatopsis* par la souche *E. coli* ET12567 + pUZ8002.

b'. amplification et clonage du promoteur ermE*

L'ajout du promoteur ermE* et du gène de résistance au chloramphénicol a été réalisé par recombinaison *in vivo* entre de courtes séquences identiques (PCR-targeting) comme décrit par Yu et al. (2000) (Proc Natl Acad Sci USA. 2000 May 23;97(11):5978-83).
Pour cela une séquence correspondant au gène de résistance au chlroramphénicol, au promoteur *ermE** et au RBS du gène *tipA* a été obtenue par PCR en utilisant comme matrice le plasmide pOSV605 et comme amorces les oligonucléotides LG001 et LG002 ayant respectivement pour séquences les séquences SEQ ID N0 : 9 et SEQ ID N0 : 10.
Ceci a permis d'obtenir un fragment présentant à chaque extrémité des séquences identiques à celles de deux régions proches sur le plasmide pLSF01b :

- l'une dans la partie du vecteur en amont du gène *ech,*
- l'autre à l'extrémité 5' du gène *ech.*

La recombinaison a eu lieu in vivo dans la souche DY330 (Yu et al. 2000) contenant le plasmide pLSF01b, après transformation par le produit PCR.
Les clones ayant intégré le promoteur ermE* ont été sélectionnés sur la base de l'acquisition de la résistance au chloramphénicol. Les clones sélectionnés sont appelés plasmides pLSF07.

c. transformation

La souche ET12567 contenant le plasmide pUZ8002 est transformée par électroporation avec le plasmide pLSF07 issu des étapes précédentes, comprenant les copies supplémentaires des gènes *ech* et *fcs* et le promoteur fort ermE* associé au RBS.

d. sélection des transformants

La sélection des transformants ayant intégré le plasmide pLSF07 se fait sur un milieu comprenant de la kanamycine.

e. Conjugaison interspécifique

Les cellules *d'Amycolatopsis* Zyl 926 sont synchronisées pour la germination par un chauffage à 50°C pendant 10 minutes.

Les transformants et les cellules *d'Amycolatopsis* Zyl 926 ainsi préparées sont ensuite mis en contact par une co-culture durant plusieurs heures pour la réalisation de la conjugaison interspécifique.

f. Sélection des souches Zyl 926 transformées

La sélection des souches *Amycolatopsis* Zyl 926 ayant intégré des copies supplémentaires des gènes *ech* et *fcs* se fait sur un milieu contenant de la kanamycine.

*Résultats*

Cette dernière étape a permis de sélectionner les souches déposées à la CNCM le 11 décembre 2014 sous les numéros I-4925, I-4926 et I-4927 ayant intégré les copies supplémentaires des gènes *ech* et *fcs* au niveau du site d'intégration du phage φBT1.

EXEMPLE 3 : Production de vanilline par la souche *Amycolatopsis* sp. I-4922

*Matériel et méthode*

Souches bactériennes :

- CNCM-I-4922
- Zyl 926

Le procédé de production de vanilline mis en oeuvre dans cet exemple est le procédé cité dans la demande EP 1 611 244 A1.

La productivité maximale est calculée selon l'équation suivante :

$$\frac{\text{Concentration en vanilline produite (g/Kg)}}{\text{Temps écoulé pour la produire (heures)}}$$

Le rendement molaire est calculé selon l'équation suivante :

$$100 \times \frac{\text{concentration molaire de vanilline}}{\text{concentration molaire d'acide férulique}}$$

Le rendement massique global vanilline/acide férulique est calculé selon l'équation suivante :

$$100 \times \frac{\text{concentration massique de vanilline}}{\text{concentration massique d'acide férulique}}$$

*Résultats*

Les résultats obtenus en utilisant le procédé de production de vanilline de la demande EP 1 611 244 avec la souche selon l'invention I-4922 et la souche Zyl 926 sont présentés dans le tableau 1 ci-dessous.

Tableau 1

|  | I-4922 | Zyl 926 (EP 1 611 244) |
|---|---|---|
| Concentration initiale en acide férulique | 25,5 g/Kg | 25 g/L |
| Productivité maximale | 0,52 g/Kg.h | Non défini |
| Résiduel d'acide férulique | 0,1 g/Kg | 1.01 g/L |
| Vanilline produite | 17,1 g/Kg | 13.8 g/L |
| Acide vanillique produit | 0,8 g/Kg | Non défini |
| Rendement molaire | 86% | 73% |
| Rendement massique global vanilline/acide férulique | 67% | 55% |
| *A noter que la densité étant proche de 1, l'unité g/Kg est équivalente à g/L.* | | |

*Conclusion*

Les résultats obtenus montrent que la souche selon l'invention I-4922 permet d'obtenir une plus grande quantité de vanilline, un rendement molaire amélioré, et la consommation de la quasi-totalité de l'acide férulique par rapport à la souche Zyl 926 de l'état de la technique.

EXEMPLE 4 : Comparaison de la production de vanilline en utilisant les souches I-4922 et I-4926

*Matériel et méthode*

Souches bactériennes :

- CNCM-I-4922 ($\varphi$C31)
- CNCM-I-4926 ($\varphi$BT1)
- Zyl 926

Le procédé de production de vanilline mis en oeuvre dans cet exemple est le procédé cité dans la demande EP 1 611 244 A1.

*Résultats*

Les résultats obtenus en utilisant le procédé de production de vanilline de la demande EP 1 611 244 avec les souches I-4922, I-4926 et Zyl 926 sont présentés dans le tableau 2 ci-dessous.

Tableau 2

|  | Zyl 926 | I-4926 | I-4922 |
|---|---|---|---|
| Concentration initiale en acide férulique | 25 g/L | 24.8 g/Kg | 25.5 g/Kg |
| Vanilline produite | 13.8 g/L | 14.4 g/Kg | 17.5 g/Kg |

*Conclusion*

Les résultats montrent que la souche selon l'invention I-4922 ayant intégré au moins une copie supplémentaire des gènes *ech* et *fcs* au niveau du site d'intégration du phage $\varphi$C31 permet de produire plus de vanilline par rapport à la souche de l'art antérieur Zyl 926.

Les résultats montrent également que la souche I-4926 ayant intégré au moins une copie supplémentaire des gènes *ech* et *fcs* au niveau du site d'intégration du phage $\varphi$BT1 n'améliore pas la production de vanilline par rapport à la souche de l'art antérieur Zyl 926.

L'intégration d'au moins une copie supplémentaire des gènes *ech* et *fcs* selon l'invention dans la souche Zyl 926 est donc locus dépendante.

**Revendications**

1. Souche *Amycolatopsis* sp. déposée auprès de la CNCM sous le numéro I-4922, I-4923, ou I-4924 le 11 décembre 2014.

2. Utilisation dans un procédé de production de vanilline d'une souche *Amycolatopsis* sp. selon la revendication 1.

3. Procédé de production de vanilline **caractérisé en ce que** la réaction de bioconversion est réalisée dans un milieu comprenant une souche *Amycolatopsis* sp. selon la revendication 1.

**Patentansprüche**

1. *Amycolatopsis* sp.-Stamm, hinterlegt bei der CNCM unter der Nummer I-4922, I-4923 oder I-4924 am 11. Dezember 2014.

2. Verwendung eines *Amycolatopsis* sp.-Stamms nach Anspruch 1 in einem Verfahren zur Herstellung von Vanillin.

3. Verfahren zur Herstellung von Vanillin, **dadurch gekennzeichnet, dass** die Biokonversionsreaktion in einem Medium durchgeführt wird, das einen *Amycolatopsis* sp.-Stamm nach Anspruch 1 umfasst.

**Claims**

1. An *Amycolatopsis* sp. strain filed with the CNCM under the number I-4922, I-4923 or I-4924 on 11 December 2014.

2. Use in a method of producing vanillin of an *Amycolatopsis* sp. strain according to claim 1.

3. A method of producing vanillin, **characterized in that** the bioconversion reaction is carried out in a medium comprising an *Amycolatopsis* sp. strain according to claim 1.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2721148 A1
- WO 2012172108 A1
- EP 1611244 A1
- EP 1611244 A

**Littérature non-brevet citée dans la description**

- **KIESER et al.** Practical Streptomyces Genetics. The John Innes Foundation, 2000
- **KERAVALA et al.** *Methods Mol Biol.,* 2008
- **BIBB et al.** *Mol Microbiol.,* Novembre 1994, vol. 14 (3), 533-45
- **BIERMAN et al.** *Gene,* 1992, vol. 116 (1), 43-49
- **MACNEIL et al.** *Gene,* 1992, vol. 111, 61-68
- **YU et al.** *Proc Natl Acad Sci USA.,* 23 Mai 2000, vol. 97 (11), 5978-83